# EUROPEAN PATENT APPLICATION

(11) **EP 1 118 662 A2**
(43) Date of publication of application: **25.07.2001**
(21) Application number: 01200163.2
(22) Date of filing: 17.01.2001
(51) Int. Cl.: C12N 15/12, C07K 14/47, C07K 16/18, G01N 33/50, G01N 33/68, A61K 38/17, A61K 48/00

(54) **Apoptin-associating protein**

(30) Priority: 17.01.2000 EP 00200169; 07.04.2000 EP 00250118
(71) Applicant: Leadd B.V., 2333 AL Leiden (NL)
(72) Inventor: Noteborn, Mathieu Hubertus Maria, 2352 EH Leiderdorp (NL); Danen-van Oorschot, Astrid Adriana Maria, 2651 VG Berkel en Rodenrijs (NL); Rohn, Jennifer Leigh, 1074 BR Amsterdam (NL); Weiss, Bertram, 14195 Berlin (DE)
(74) Representative: Prins, Adrianus Willem

(57) **Abstract**

The invention relates to the field of apoptosis. The invention provides novel therapeutic possibilities, for example novel combinatorial therapies or novel therapeutic compounds that can work alone, sequentially to, or jointly with Apoptin, especially in those cases wherein p53 is (partly) non-functional.

## Description

The invention relates to the field of apoptosis. Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighboring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).
The apoptotic process can be initiated by a variety of regulatory stimuli (Wyllie, 1995, White 1996, Levine, 1997). Changes in the cell survival rate play an important role in human pathogenesis of diseases, e.g. in cancer development and auto-immune diseases, where enhanced proliferation or decreased cell death (Kerr et al., 1994, Paulovich, 1997) is observed. A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).
Many tumors, however, acquire a mutation in p53 during their development, often correlating with poor response to cancer therapy. Certain transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).
For such tumors lacking functional p53 (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or can not be blocked by anti-apoptotic activities, such as Bcl-2 or Bcr-abl-like ones. These factors might be part of a distinct apoptosis pathway or might be (far) downstream of the apoptosis inhibiting compounds.
Apoptin is a small protein derived from chicken anemia virus (CAV; Noteborn and De Boer, 1995, Noteborn et al., 1991, Noteborn et al., 1994; 1998a), which can induce apoptosis in human malignant and transformed cell lines, but not in untransformed human cell cultures. *In vitro,* Apoptin fails to induce programmed cell death in normal lymphoid, dermal, epidermal, endothelial and smooth-muscle cells. However, when normal cells are transformed they become susceptible to apoptosis by Apoptin. Long-term expression of Apoptin in normal human fibroblasts revealed that Apoptin has no toxic or transforming activity in these cells (Danen-van Oorschot, 1997 and Noteborn, 1996).
In normal cells, Apoptin was found predominantly in the cytoplasm, whereas in transformed or malignant cells i.e. characterized by hyperplasia, metaplasia or dysplasia, it was located in the nucleus, suggesting that the localization of Apoptin is related to its activity (Danen-van Oorschot et al. 1997).
Apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn, 1996).
Therefore, Apoptin is a therapeutic compound for the selective destruction of tumor cells, or other hyperplasia, metaplasia or dysplasia, especially for those tumor cells which have become resistant to (chemo)-therapeutic induction of apoptosis, due to the lack of functional p53 and (over)-expression of Bcl-2 and other apoptosis-inhibiting agents (Noteborn and Pietersen, 1998). It appears, that even pre-malignant, minimally transformed cells, are sensitive to the death-inducing effect of Apoptin. In addition, Noteborn and Zhang (1998) have shown that Apoptin-induced apoptosis can be used as diagnosis of cancer-prone cells and treatment of cancer-prone cells.
The fact that Apoptin does not induce apoptosis in normal human cells, at least not *in vitro,* shows that a toxic effect of Apoptin treatment *in vivo* will be very low. Noteborn and Pietersen (1998) and Pietersen et al. (1999) have provided evidence that adenovirus expressed Apoptin does not have an acute toxic effect *in vivo.* In addition, in nude mice it was shown that Apoptin has a strong anti-tumor activity.
However, to further enlarge the array of therapeutic anti-cancer or anti-auto-immune-disease compounds available in the art, additional therapeutic compounds are desired that are designed to work alone, sequentially to, or jointly with Apoptin, especially in those cases wherein p53 is (partly) non-functional.
The invention provides novel therapeutic possibilities, for example novel combinatorial therapies or novel therapeutic compounds that can work alone, sequentially to, or jointly with Apoptin, especially in those cases wherein p53 is (partly) non-functional.

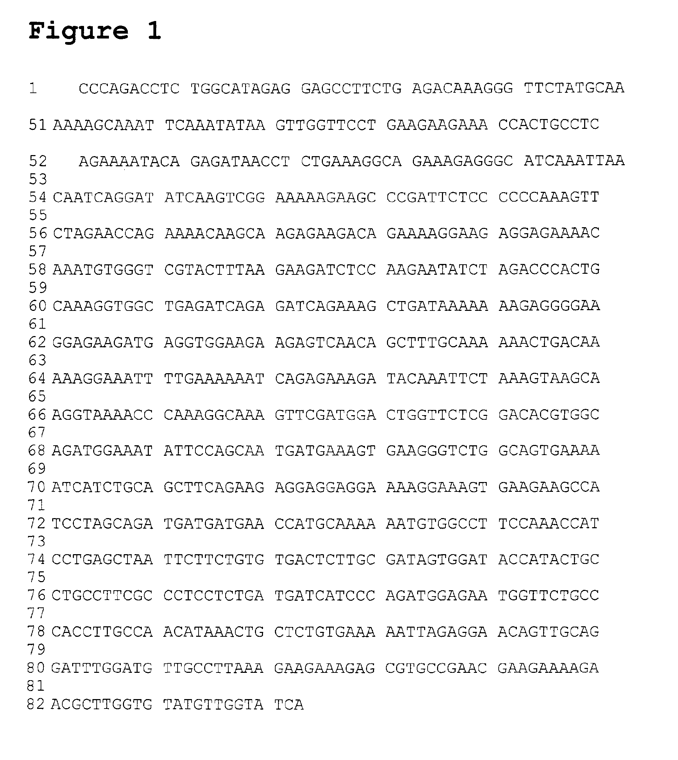

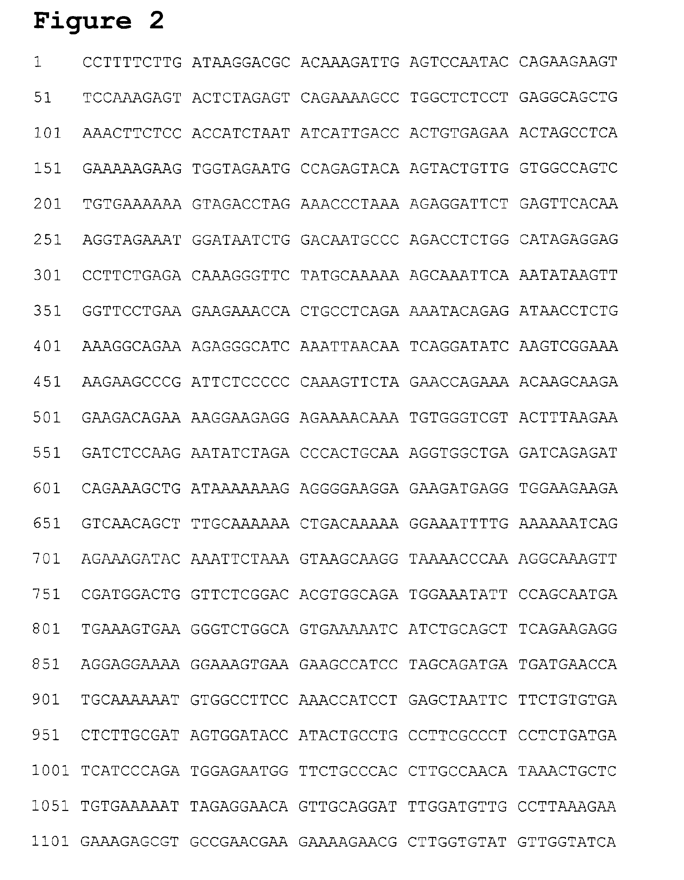

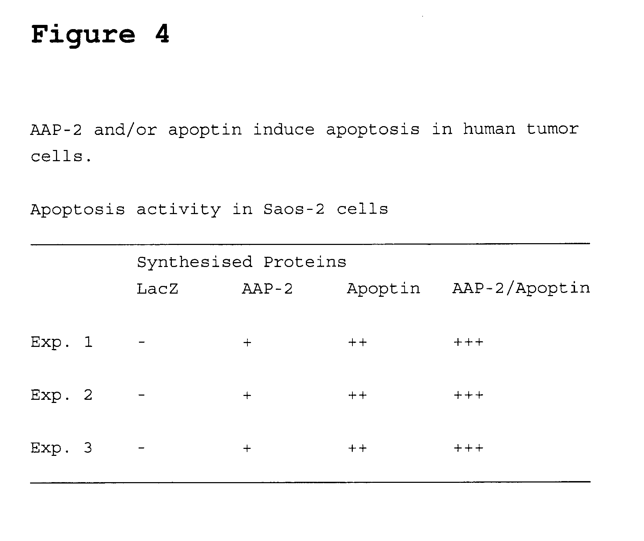

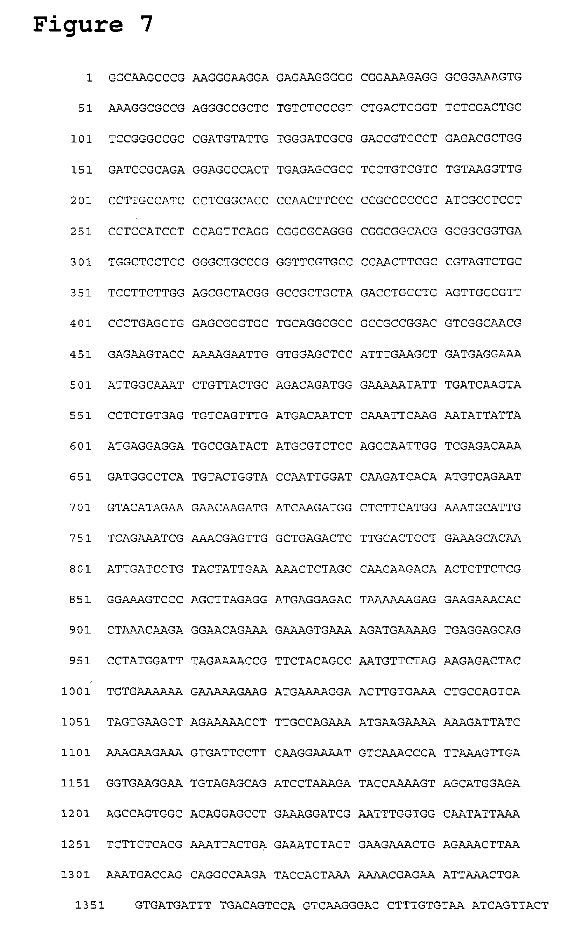

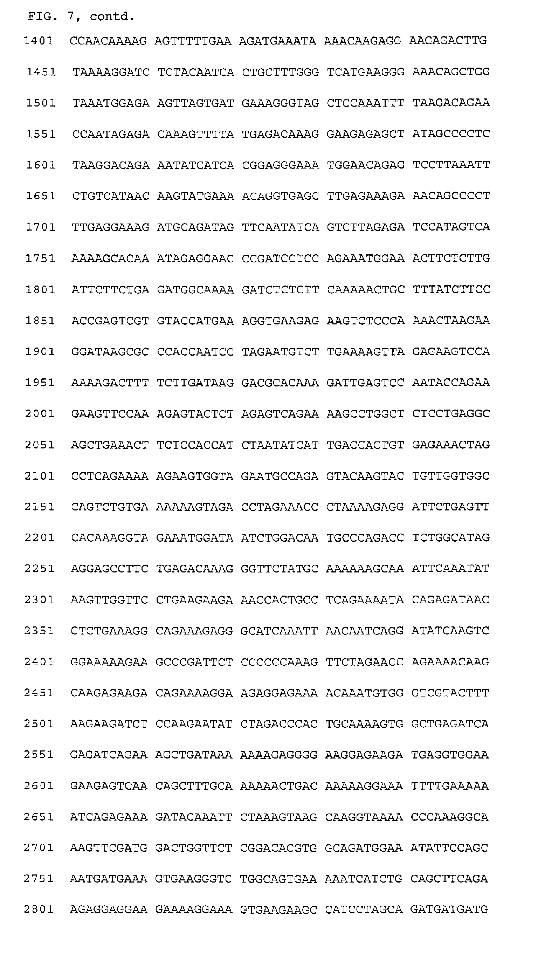

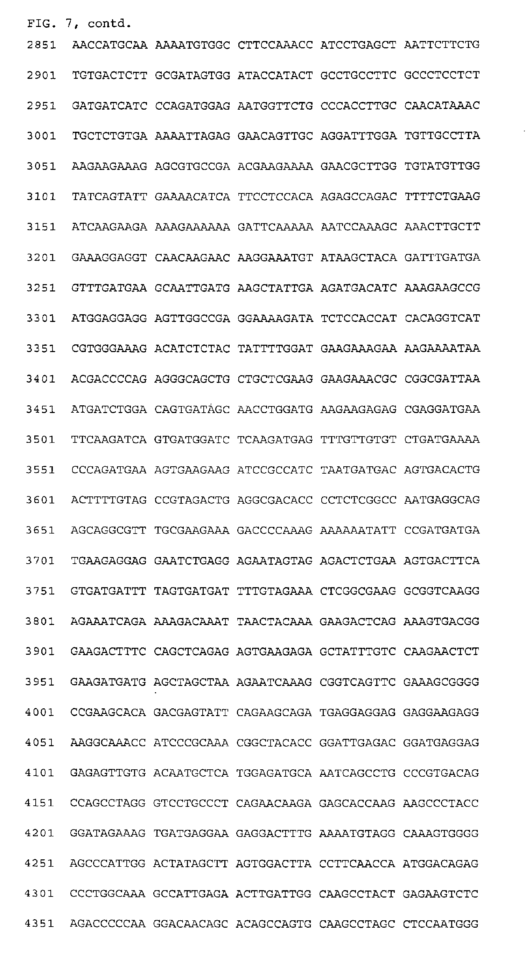

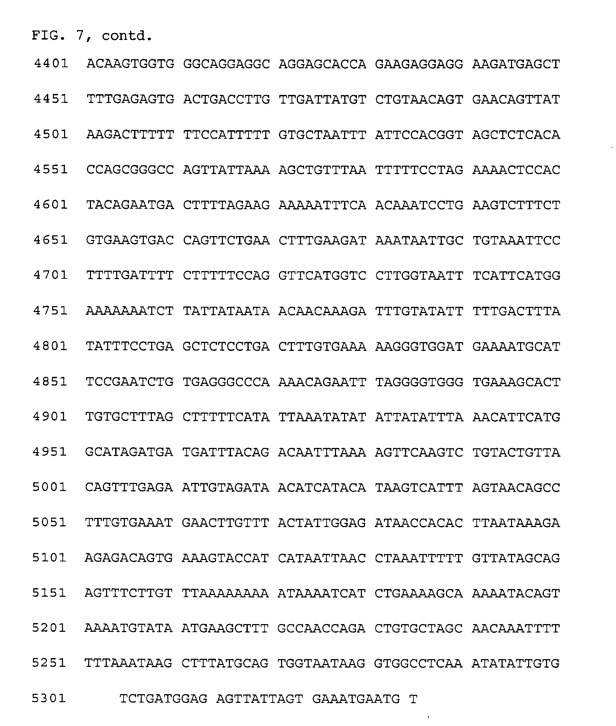

In a first embodiment, the invention provides an isolated or recombinant nucleic acid or functional equivalent or fragment thereof encoding an Apoptin-associating proteinaceous substance capable of providing apoptosis, alone or in combination with other apoptosis inducing substances, such as Apoptin. Proteinaceous substance herein is defined as a substance comprising a peptide, polypeptide or protein, optionally having been modified by for example glycosylation, myristilation, phosphorylation, the addition of lipids, by homologous or heterologous di- or multi-merisation, or any other (posttranslational) modifications known in the art.
Apoptin-associating herein is defined as belonging to the cascade of substances specifically involved in the cascade of events found in the apoptosis pathway as inducable by Apoptin, preferably those substances that are specifically involved in the p53-independent apoptosis pathway.
In a preferred embodiment, the invention provides an isolated or recombinant nucleic acid or functional equivalent or fragment thereof encoding an Apoptin-associating proteinaceous substance capable of providing apoptosis derived from a cDNA library, preferably a vertebrate cDNA library, such as derivable from poultry, but more preferably a mammalian cDNA library, preferably wherein said cDNA library comprises human cDNA. An Apoptin-associating proteinaceous substance obtained by determining a vertebrate analogue (preferably human) of an Apoptin-associating proteinaceous substance derived from an invertebrate cDNA library is also included.
In another embodiment, the invention provides an isolated or recombinant nucleic acid or functional equivalent or fragment thereof encoding an Apoptin-associating proteinaceous substance capable of providing apoptosis capable of hybridising to a nucleic acid molecule encoding an Apoptin-associating proteinaceous substance capable of providing apoptosis as shown in figure 1, 2, 5 and/or 7, in particular encoding a novel protein capable of providing apoptosis or functional equivalent or functional fragment thereof called Apoptin-associating protein 2 or 3, abbreviated herein also as AAP-2 or AAP-3. Figure 1 and 2 show an approximately 1100 and 900 bp fragment of the AAP-2 fragment as depicted in Figure 7. All 3 nucleotide sequences encode an protein with at least the capability of binding to Apoptin and providing apoptosis. Of course, an isolated or recombinant nucleic acid or functional equivalent or fragment thereof encoding an additional Apoptin-associating proteinaceous substance capable of associating with the AAP-2 or AAP-3 protein are herewith also provided, means and methods to arrive at such an additional protein located in the Apoptin cascade follow those of the detailed description given herein. Knowledge derived from studying the AAP-2 and/or AAP-3 clones is exploited to determine a functional pathway in which AAP-2 and/or AAP-3 is/are involved, thus allowing the design of a therapeutic means of intervening in such a pathway.
A functional equivalent or a functional fragment thereof is a derivative or a fragment having the same kind of activity possibly in different amounts. It is clear to a person skilled in the art that there are different ways of arriving at a functional equivalent or functional fragment. A functional equivalent can for example be a point mutant or a deletion mutant or a equivalent derived from another species. Another way to arrive at a functional equivalent is a molecular evolution of equivalents and/or fragments having the same kind of activity possibly in different amounts.
In particular, the invention provides an isolated or recombinant nucleic acid or functional equivalent or fragment thereof encoding an Apoptin-associating proteinaceous substance capable of providing apoptosis being at least 60% homologous, preferably at least 70%, more preferably at least 80%, even more preferably 90% and most preferably at least 95% homologous to a nucleic acid molecule, or to a functional equivalent or functional fragment thereof, encoding an Apoptin-associating proteinaceous substance as shown in figure 1, 2, 5 or 7.
Furthermore, the invention provides a vector comprising a nucleic acid according to the invention. Examples of such a vector are given in the detailed description given herein; such as vector pMT2SM-AAP-2 or -AAP-3, pMT2SM vector expressing Myc-tagged AAP-2 or AAP-3 cDNA, a plasmid expressing an Apoptin-associating protein fragment, and so on. These and other vectors are for example useful in finding additional Apoptin-associating proteinaceous substances from the cascade, as defined above.
In yet another embodiment, the invention provides a vector comprising a nucleic acid according to the invention, said vector comprising a gene-delivery vehicle, making the invention very useful in gene therapy. By equipping a gene delivery vehicle with a nucleic acid according to the invention, and by targeting said vehicle to a cell or cells that have been over-proliferating and/or have shown decreased death rates, said gene delivery vehicle provides said cell or cells with the necessary means for apoptosis, providing far reaching therapeutic possibilities.
Furthermore, the invention provides a host cell comprising a nucleic acid or a vector according to the invention. Examples comprise transformed or transfected bacterial or yeast cells as described in the detailed description herein. Preferred is a host cell according to the invention which is a transformed eukaryotic cell such as a yeast cell or a vertebrate cell, such as mammalian or Cos cells transformed or transfected with a nucleic acid or vector according to the invention. Said cells are in general capable to express or produce a proteinaceous substance capable of providing apoptosis with the ability to associate with Apoptin.
The invention furthermore provides an isolated or recombinant Apoptin-associating proteinaceous substance capable of providing apoptosis. As for example shown herein in figure 4, expression of such Apoptin-associating proteinaceous substance in cells, such as tumour cells, or other over-proliferating cells, induces the apoptic process. It can do so alone, or in the presence of other apoptosis inducing substances such as Apoptin, and especially so independent of p53, showing that also in those cases where (functional) p53 is absent apoptosis can be induced by a substance according to the invention. In particular, the invention provides a proteinaceous substance according to the invention encoded by a nucleic acid according to the invention, for example comprising at least a part of an amino acid sequence as shown in figure 4 or a functional equivalent or functional fragment thereof capable of providing apoptosis alone or in combination with other apoptosis inducing substances such as Apoptin.
The invention also provides an isolated or synthetic antibody specifically recognising a proteinaceous substance or functional equivalent or functional fragment thereof according to the invention. Examples of such an antibody are given in the detailed description given herein. Such an antibody is for example obtainable by immunising an experimental animal with a Apoptin-associating proteinaceous substance or an immunogenic fragment or equivalent thereof and harvesting polyclonal antibodies from said immunised animal (as shown herein in the detailed description), or obtainable by other methods known in the art such as by producing monoclonal antibodies, or (single chain) antibodies or binding proteins expressed from recombinant nucleic acid derived from a nucleic acid library, for example obtainable via phage display techniques.
With such an antibody, the invention also provides a proteinaceous substance specifically recognisable by such an antibody according to the invention, for example obtainable via immunoprecipitation, Western Blotting, or other immunological techniques known in the art.
Furthermore, the invention provides use of a nucleic acid, vector, host cell, or proteinaceous substance according to the invention for the induction of tumor-specific apoptosis, as for example shown in figure 4. In particular, such use is provided wherein said apoptosis is p53-independent. In particular, such use is also provided further comprising use of a nucleic acid encoding Apoptin or a functional equivalent or fragment thereof or use of Apoptin or a functional equivalent or fragment thereof. As can be seen from figure 4, combining these Apoptin-inducing substances increases the percentage apoptosis of treated tumour cells.
Such use as provided by the invention is particularly useful from a therapeutic viewpoint. The invention provides herewith a pharmaceutical composition comprising a nucleic acid, vector, host cell, or proteinaceous substance according to the invention. In addition, such a pharmaceutical composition according to the invention is provided further comprising a nucleic acid encoding Apoptin or a functional equivalent or fragment thereof.
Such a pharmaceutical composition is in particular provided for the induction of apoptosis, for example wherein said apoptosis is p53-independent, for the treatment of a disease where enhanced cell proliferation or decreased cell death is observed, as is in general the case when said disease comprises cancer or auto-immune disease. Herewith the invention provides a method for treating an individual carrying a disease where enhanced cell proliferation or decreased cell death is observed comprising treating said individual with a pharmaceutical composition according to the invention. In particular these compositions comprise a factor of an apoptosis pathway, which is specific for transformed cells. Therefore, these compositions are essential for new treatments, but also for diagnosis of diseases related with aberrance's in the apoptotic process, such as cancer, cancer-proneness and autoimmune diseases.
Furthermore, the invention provides a diagnostic assay based on the tumor-specific nuclear localization behavior of AAP-2 such as its dominant nuclear localization in human tumor cells but not in normal healthy cells.
The invention also provides an isolated or recombinant nucleic acid as set forth in SEQ ID No 6. An isolated or recombinant proteinaceous substance encoded by a nucleic acid as set forth in SEQ ID No 6 is also included.
The invention provides an isolated or recombinant proteinaceous substance comprising the amino acid sequence as set forth in SEQ ID No 7.
The invention also provides an isolated or recombinant proteinaceous substance comprising the amino acid sequence as set forth in SEQ ID No 8.
In a further embodiment the invention provides an assay to identify a putative effector of the activity of the proteinaceous substance encoded by a nucleic acid as set forth in SEQ ID No 6 comprising bringing in contact a proteinaceous substance comprising the amino acid sequence as set forth in SEQ ID No 8 with said effector and determining the binding of said effector. Examples of methods to arrive at such an effector are binding studies, where an AAP-2 peptide comprising the PHD-finger domain (SEQ ID No 8) is bound to a matrix and is tested whether test substances bind to the AAP-2 peptide, or by co-immunoprecipitation of an AAP-2 peptide comprising the PHD-finger domain with test substances using antibodies generated against the AAP-2 peptide comprising the PHD-finger domain. Test substances are for example small organic compounds derived e.g. from a compound library or peptides or proteins derived e.g. from a peptide library or from a natural source like a cell extract. The test substances are for example labeled for easier detection. The substances found to bind to the PHD-finger domain may either enhance or inhibit one or more effects of AAP-2. This is, for example, tested by measuring the apoptotic activity of AAP-2 as described above in the presence of said substances and by determining the nuclear localization of AAP-2 as described above in the presence of said substances. It is clear to a person skilled in the art that an assay to identify a putative effector of the activity of a proteinaceous substance encoded by a nucleic acid as set forth in SEQ ID No 6 can also be performed with a functional equivalent or a functional fragment of SEQ ID No 8 having the same kind of activity possibly in different amounts.

The invention will be explained in more detail in the following detailed description, which is not limiting the invention.

### Detailed description

We have used the yeast-2 hybrid system (Durfee et al., 1993) to identify Apoptin-associating cellular compounds, which are essential in the induction of apoptosis. The used system is an *in vivo* strategy to identify human proteins capable of physically associating with Apoptin. It has been used to screen cDNA libraries for clones encoding proteins capable of binding to a protein of interest (Fields and Song, 1989, Yang et al., 1992). The invention provides a for example novel Apoptin-associating protein, which is named Apoptin-associating protein 2 abbreviated as AAP-2. The invention also provides a method for inducing apoptosis through interference with the function of this newly discovered AAP-2 protein or functional equivalents or fragments thereof and/or the induction of apoptosis by means of (over)expression of AAP-2 or related gene or functional equivalents or fragments thereof. In addition, the invention also provides another Apoptin-associating protein, named AAP-3.
The invention also provides an anti-tumor therapy based on the interference with the function of AAP-2-like proteins and/or its (over)expression. An aberrant high level of AAP-2-like proteins will result in the induction of the opposite process of cell transformation, namely apoptosis. The invention furthermore provides a mediator of Apoptin-induced apoptosis, which is tumor-specific. The invention provides a therapy for cancer, autoimmune diseases or related diseases which is based on AAP-2-like proteins alone or in combination with Apoptin and/or Apoptin-like compounds.

### Construction of pGBT9-VP3

For the construction of the bait plasmid, which enables the identification of Apoptin-associating proteins by means of a yeast-two-hybrid system, plasmid pET-16b-VP3 (Noteborn, unpublished results) was treated with *Nde*I and *BamH*I. The 0.4 kb *Nde*I-*BamH*I DNA fragment was isolated from low-melting-point agarose.
Plasmid pGBT9 (Clontech Laboratories, Inc, Palo Alto, USA) was treated with the restriction enzymes *EcoR*I and *BamH*I. The about 5.4-kb DNA fragment was isolated and ligated to an *EcoR*I*-Nde*I linker and the 0.4-kb DNA fragment containing the Apoptin-encoding sequences starting from its own ATG-initiation codon. The final construct containing a fusion gene of the GAL4-binding domain sequence and Apoptin under the regulation of the yeast promoter ADH was called pGBT-VP3 and was proven to be correct by restriction-enzyme analysis and DNA-sequencing according to the Sanger method (1977).
All cloning steps were essentially carried out as described by Maniatis et al. (1992). The plasmid pGBT-VP3 was purified by centrifugation in a CsCl gradient and column chromatography in Sephacryl S500 (Pharmacia).

### GAL4-activation domain-tagged cDNA library

The expression vector pACT, containing the cDNAs from Epstein-Barr-virus-transformed human B cells fused to the GAL4 transcriptional activation domain, was used for detecting Apoptin-associating proteins. The pACT c-DNA library is derived from the lambda-ACT cDNA library, as described by Durfee et al. 1993.

### Bacterial and Yeast strains

The *E.coli* strain JM109 was the transformation recipient for the plasmid pGBT9 and pGBT-VP3. The bacterial strain electromax/DH10B was used for the transformation needed for the recovery of the Apoptin-associating pACT-cDNAs, and was obtained from GIBCO-BRL, USA.
The yeast strain Y190 was used for screening the cDNA library, and all other transformations, which are part of the used yeast-two-hybrid system.

### Media

For drug selections Luria Broth (LB) plates for *E.coli* were supplemented with ampicillin (50 microgram per ml). Yeast YPD and SC media were prepared as described by Rose et al. (1990).

### Transformation of competent yeast strain Y190 with plasmids pGBT-VP3 and pACT-cDNA and screening for beta-galactosidase activity.

The yeast strain Y190 was made competent and transformed according to the methods described by Klebe et al. (1983). The yeast cells were first transformed with pGBT-VP3 and subsequently transformed with pACT-cDNA, and these transformed yeast cells were grown on histidine-minus plates, also lacking leucine and tryptophan.
Hybond-N filters were layed on yeast colonies, which were histidine-positive and allowed to wet completely. The filters were lifted and submerged in liquid nitrogen to permeabilize the yeast cells. The filters were thawed and layed with the colony side up on Whattman 3MM paper in a petridish with Z-buffer (Per liter: 16.1 gr Na₂HPO₄.7H₂O, 5.5 gr NaH₂PO₄.H₂O, 0.75 gr KCl and 0,246 gr MgSO₄.7H₂O, pH 7.0) containing 0.27% beta-mercapto-ethanol and 1 mg/ml X-gal. The filters were incubated for at least 15 minutes or during night.

### Recovery of plasmids from yeast

Total DNA from yeast cells, which were histidine- and beta-galactosidase-positive, was prepared by using the glusulase-alkaline lysis method as described by Hoffman and Winston (1987) and used to transform Electromax/DH10B bacteria via electroporation using a Bio-Rad GenePulser according the manufacturer's specifications.

Transformants were plated on LB media containing the antibiotic agent ampicillin.

### Isolation of Apoptin-associating pACT clones

By means of colony-filter assay the colonies were lysed and hybridized to a radioactive-labeled 17-mer oligomer, which is specific for pACT (see also section Sequence analysis). Plasmid DNA was isolated from the pACT-clones, and by means of *Xho*I digestion analysed for the presence of a cDNA insert.

### Sequence analysis

The subclone containing the sequence encoding Apoptin-associating protein was partially sequenced using dideoxy NTPs according to the Sanger-method, which was performed by Eurogentec, Seraing, Belgium). The used sequencing primer was a pACT-specific 17-mer comprising of the DNA-sequence 5'-TACCACTACAATGGATG-3'.
The sequences of the Apoptin-associating cDNAs were compared with known gene sequences from the EMBL/Genbank.

### Generation and testing of antibodies.

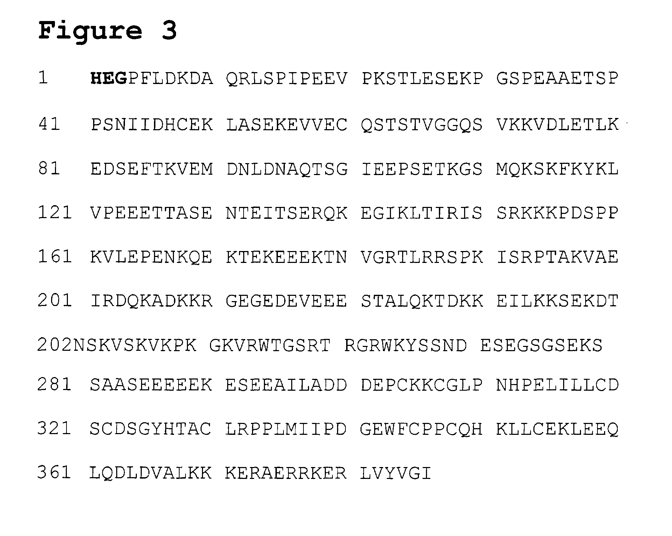

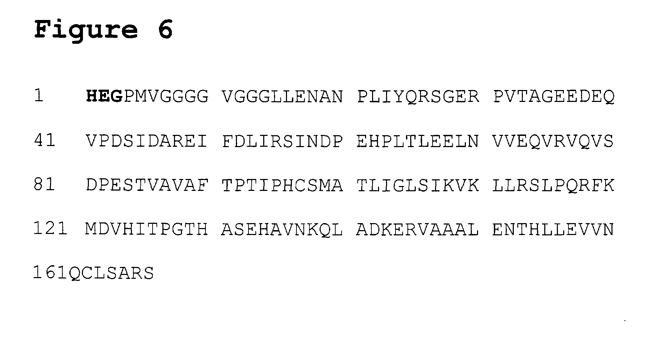

In order to generate polyclonal antisera against the AAP-2 and AAP-3 protein, we designed three peptides per protein. These peptides were for AAP-2:
1) EVPKSTLESEKPGSP (19-33)
2) ISSRKKKPDSPPKVL (149-163)
3) TGSRTRGRWKYSSND (256-270)
The peptides for AAP-3 were:
IYQRSGERPVTAGEE (23-37)
DEQVPDSIDAREIFD (38-52)
RSINDPEHPLTLEEL (55-69)
The numbers in parenthesis correspond respectively to the amino acid sequence of Figure 3 and 6.

These peptides were synthesized at Eurogentec (Belgium) with the standard addition of a C- terminal or N-terminal cysteine residue and all subsequent antibody syntheses was also performed there. These peptides were coupled to Keyhole Limpet Hemocyanin (KLH) and injected as a cocktail into two separate specific pathogen free rabbits with an immunization schedule of one injection and three subsequent boosts. Blood samples were taken before and after immunization. The sera were tested in-house for specific reactivity to the peptide cocktail by ELISA. The titers from each rabbit were high (>200,000). Furthermore, for certain subsequent purposes, the AAP-2 and AAP-3 antibodies were immune-purified using peptide cocktail coupled to immobilized diaminodipropylamine agarose columns (Pierce) according to the manufacturer's protocol.
The best AAP-2 and AAP-3 antibody preparation of the two generated was selected for further use. We tested the efficacy of this antibody by transfecting 6 cm plates of sub-confluent primate COS-7 and human U₂OS cells using the calcium phosphate co-precipitation method with 5 µg of the AAP-2-myc or AAP-3-myc plasmid DNA construct, and as a control, untransfected cells. Two days post-transfection, cells were washed briefly in PBS, lysed in RIPA buffer (10 mM Tris 7.5, 150 mM NaCl, 0.1% SDS, 1.0% NP-49 and 1.0 % sodium deoxycholate), clarified by centrifugation, and the supernatant fractionated on SDS-denaturing polyacrylamide gel electrophoresis. Proteins were Western-transferred to PVDF membranes (Immobilon, Millipore) using standard methodology. Membranes were blocked in 5% non-fat dry milk in tris-buffered saline containing 0.1% Tween-20, then incubated in the unpurified AAP-2 or AAP-3 antisera at a concentration of 1:5000. After a brief wash, membranes were further incubated in HRP-conjugated goat-anti-rabbit Ig at a concentration of 1:2000. After a thorough series of wash steps, proteins were detected using enhanced chemiluminescence (Amersham) according to the manufacturer's protocol, and exposed to x-ray film and developed using standard automated machinery.
In addition, we tested the purified AAP-2 and AAP-3 antibody using immunoprecipitation in a manner the same as above, except that after centrifugation, the supernatant was added to 10 ul of AAP-2 or AAP-3 antibody pre-coupled to protein-A-sepharose beads, incubated for 1 hour with tumbling, then washed before fractionation on SDS-PAGE gels and Western analysis. Detection in this case was performed with the anti-myc tag monoclonal antibody 9E10 (Evan et al. 1985).
Finally, the purified antibody was tested for utility in immunofluorescence by including glass coverslips in the above transfections. Coverslips were fixed with 4% paraformaldehyde, blocked with normal goat serum, incubated in AAP-2 or AAP-3 antibody diluted 1:5, washed, incubated in FITC-conjugated goat-anti-rabbit Ig, mounted and visualized under fluorescence microscopy.

### Results and discussion

Apoptin induces specifically apoptosis in transformed cells, such as cell lines derived from human tumors. To identify the essential compounds in this cell-transformation-specific and/or tumor-specific apoptosis pathway, a yeast genetic screen was carried out.
We have used a human cDNA library, which is based on the plasmid vector pACT containing the complete cDNA copies made from Epstein-Barr virus-transformed human B cells (Durfee et al., 1993).

### Construction of a bait plasmid expressing a fusion gene product of GAL4-DNA-binding domain and Apoptin

To examine the existence of Apoptin-associating proteins in the human transformed/tumorigenic cDNA library, a so-called bait plasmid had to be constructed. To that end, the complete Apoptin-encoding region, flanked by about 40 basepairs downstream from the Apoptin gene, was cloned in the multiple cloning site of plasmid pGBT9.
The final construct, called pGBT-VP3, was analyzed by restriction-enzyme analysis and sequencing of the fusion area between Apoptin and the GAL4-DNA-binding domain.

### A gene(fragment) encoding an Apoptin-associating protein is determined by transactivation of a GAL4-responsive promoter in yeast.

The Apoptin gene is fused to the GAL4-DNA-binding domain of plasmid pGBT-VP3, whereas all cDNAs derived from the transformed human B cells are fused to the GAL4-activation domain of plasmid pACT. If one of the proteinaceous substances encoded by said cDNAs binds to Apoptin, the GAL4-DNA-binding domain will be in the vicinity of the GAL4-activation domain resulting in the activation of the GAL4-responsive promoter, which regulates the reporter genes HIS3 and LacZ.
The yeast clones containing plasmid expressing Apoptin and a plasmid expressing an Apoptin-associating protein fragment can grow on a histidine-minus medium and will stain blue in a beta-galactosidase assay. Subsequently, the plasmid with the cDNA insert encoding the Apoptin-associating protein can be isolated and characterized.
Before we could do so, however, we have determined that transformation of yeast cells with pGBT-VP3 plasmid alone, or in combination with an empty pACT vector, did not result in the activation of the GAL4-responsive promoter.

### Identification of Apoptin-associating protein encoded by cDNA derived from a human transformed B cell line.

We have found two independent yeast colonies, which upon transformation with pGBT-VP3 and pACT-cDNA were able to grow on a histidine-minus medium (also lacking leucine and tryptophan) and stained blue in a beta-galactosidase assay. These results indicate that the observed yeast colonies contain besides the bait plasmid pGBT-VP3 also a pACT plasmid encoding a potential Apoptin-associating protein.
Plasmid DNA was isolated from the positive yeast colony, which was transformed in bacteria. By means of a filter-hybridization assay using a pACT-specific labeled DNA-probe, 2 independent clones containing pACT plasmid could be determined. Subsequently, pACT DNA was isolated and digested with restriction enzyme *Xho*I, which resulted in the presence of a 1.1-kbp (clone I) and a 1.3-kbp (clone II) cDNA insert, respectively. Finally, the pACT plasmids containing the two independent cDNA inserts were partially sequenced by using the Sanger method (Sanger et al., 1977).

### Description of Apoptin-associating proteins

The yeast genetic screen for Apoptin-associating proteins resulted in the detection of two cDNA clones comprising a single type of protein, namely a novel protein called Apoptin-associating protein 2, abbreviated as AAP-2.
The determined DNA sequence part of the AAP-2 cDNA clones AAP-2-I and AAP-2-II are shown in Figure 1 and 2, respectively. The amino acid sequence, derived from the detected DNA sequence of clone AAP-2-II is given in Figure 3. Below the experiments will be described for AAP-2-II, which will be referred as AAP-2.

### Construction of an expression vector for the identification of AAP-2 protein in mammalian cells.

To study whether the cloned cDNA AAP-2 indeed encode (Apoptin-associating) a protein product, we have carried out the following experiments.
The DNA plasmid pMT2SM contains the adenovirus 5 major late promoter (MLP) and the SV40 ori enabling high levels of expression of foreign genes in transformed mammalian cells, such as SV-40-transformed Cos cells.
Furthermore, the pMT2SM vector contains a Myc-tag (amino acids:
EQKLISEEDL) which is in frame with the foreign-gene product. This Myc-tag enables the recognition of e.g. Apoptin-associating proteins by means of the Myc-tag-specific 9E10 antibody.
The pMT2SM vector expressing Myc-tagged AAP-2 cDNA was constructed as follows. The pACT-AAP-2 cDNA clone was digested with the restriction enzyme *Xho*I and the cDNA insert was isolated. The expression vector pMT2SM was digested with *Xho*I and treated with calf intestine alkaline phosphatase and ligated to the isolated AAP-2 cDNA inserts. By sequence analysis, the pMT2SM constructs containing the AAP-2 cDNA in the correct orientation was identified.
The synthesis of Myc-tagged AAP-2 protein was analyzed by transfection of Cos cells with plasmid pMT2SM-AAP-2. As negative control, Cos cells were mock-transfected. Two days after transfection, the cells were lysed and Western-blot analysis was carried out using the Myc-tag-specific antibody 9E10.
The Cos cells transfected with pMT2SM-AAP-2 were proven to synthesise a specific Myc-tagged AAP-2 product with the size of approximately 70 kDa. As expected, the lysates of the mock-transfected Cos cells did not contain a protein product reacting with the Myc-tag-specific antibodies.
These results indicate that we have been able to isolate a cDNA that is able to produce a protein product with the ability to associate to the apoptosis-inducing protein Apoptin.

### Co-immunoprecipitation of Myc-tagged AAP-2 protein with Apoptin in a transformed mammalian cell system.

Next, we have analyzed the association of Apoptin and the AAP-2 protein by means of co-immunoprecipitations using the Myc-tag-specific antibody 9E10. The 9E10 antibodies were shown not to bind directly to Apoptin, which enables the use of 9E10 for carrying out co-immunoprecipitations with (myc-tagged) Apoptin-associating proteins and Apoptin.
To that end, Cos cells were co-transfected with plasmid pCMV-VP3 encoding Apoptin and with plasmid pMT2SM-AAP-2. As a negative control, cells were transfected with pCMV-VP3 expressing Apoptin and a plasmid pcDNA3.1.LacZ-myc/His-LacZ encoding the myc-tagged beta-galactosidase, which does not associate with Apoptin.

Two days after transfection, the cells were lysed in a buffer consisting of 50 mM Tris (7.5), 250 mM NaCl, 5 mM EDTA, 0.1 % Triton X100, 1 mg/ml Na₄P₂O₇ and freshly added protease inhibitors such as PMSF, Trypsine-inhibitor, Leupeptine and Na₃VO₄. The specific proteins were immuno-precipitated as described by Noteborn et al. (1998) using the Myc-tag-specific antibodies 9E10, and analyzed by Western blotting.

Staining of the Western blot with 9E10 antibodies and 111.3 antibodies, which are specifically directed against myc-tag and Apoptin, respectively, showed that the "total" cell lysates contained the 16-kDa Apoptin product and the Myc-tagged AAP-2 protein. By means of a specific LacZ polyclonal antibody also the beta-galactosidase product could be visualized.
Immunoprecipitation of the Myc-tagged AAP-2 products was accompanied by the immunoprecipatation of Apoptin product of 16 kDa. In contrast, immunoprecipitation of myc-tagged beta-galactosidase did not result in a detectable co-precipitation of the Apoptin protein. In addition, immunoprecipitation of the Apoptin protein, by means of a polyclonal antibody directed against the C-terminal part of Apoptin (Noteborn and Danen, unpublished results), was accompanied by the immunoprecipitation of the AAP-2 product of approximately 70-kDa, but not by beta-galactosidase protein.
In total, three independent immunoprecipitation experiments were carried out, which all showed the specific associating ability of Apoptin protein to the AAP-2 protein.
These results indicate that the novel determined AAP-2 protein is able to specifically associate with Apoptin not only in the yeast background, but also in a mammalian transformed cellular system.

### Over-expression of the novel AAP-2 protein in human transformed cells induces the apoptotic process.

In addition, we have examined whether AAP-2 carries apoptotic activity. First, we have analyzed the cellular localization of the novel AAP-2 protein in human transformed cells. To that end, the human osteosarcoma-derived Saos-2 cells were transfected, as described by Danen-van Oorschot (1997), with plasmid pMT2SM-AAP-2 encoding the myc-tagged AAP-2 protein, respectively.
By indirect immunofluorescence using the myc-tag-specific antibody 9E10 and DAPI, which stains the nuclear DNA, it was shown that AAP-2 protein was mainly present in the nucleus of most of the tumor cells and in a minor part of the cells both in the nucleus and cytoplasm or cytoplasm alone. These features suggest that, at least in human tumor cells, AAP-2 is involved in nuclear transport processes.
Already, three days after transfection a significant amount of Saos-2 cells synthesizing AAP-2 underwent induction of apoptosis. These AAP-2-positive cells were aberrantly stained with DAPI, which is indicative for induction of apoptosis (Telford, 1992, Danen-van Oorschot, 1997). Cells expressing Apoptin also underwent apoptosis, whereas as expected the cells synthesizing the non-apoptotic beta-galactosidase (LacZ) protein did not. Co-expression of Apoptin and AAP-2 protein in human tumor cells, such as Saos-2 cells, results in a slightly faster apoptotic process as expression of Apoptin or AAP-2 protein alone. The results are shown in Figure 4.
The fact that AAP-2 protein can induce apoptosis in p53-minus Saos-2 cells indicates that AAP-2 can induce p53-independent apoptosis. These results imply that AAP-2 can be used as anti-tumor agent in cases where other (chemo)therapeutic agents will fail. Furthermore, the finding that both Apoptin and AAP-2 induce a p53-independent pathway indicates that AAP-2 fits in the Apoptin-induced apoptotic pathway.

In conclusion, we have identified an Apoptin-associating protein, namely the novel AAP-2 protein, which is mainly present in the nucleus and able to induce (p53-independent) apoptosis in human tumor cells.

### AAP-2 does not induce apoptosis in human normal diploid cells.

Next, we have examined whether AAP-2 behaves similar in normal human diploid non-transformed cells as has been found for AAP-2 in human tumor cells.
To that end, human diploid VH10 fibroblasts (Danen-Van Oorschot, 1997) were transfected using Fugene according the protocol of the supplier (Roche, Almere, The Netherlands), with plasmid pMT2SM-AAP-2b encoding the myc-tagged AAP protein. In parallel, human tumor-derived Saos-2 cells were also transfected with plasmid pMT2SM-AAP-2. Three days after transfection, the cells were harvested and analyzed by indirect immunofluorescence using the myc-tag-specific antibody 9E10. Within the majority of AAP-2-positive human diploid cells, AAP-2 is located in the cytoplasm only or both in the nucleus and cytoplasm. As expected, in most of the human tumor Saos-2 cells, AAP-2 is only located in the nucleus. Furthermore, the AAP-2-positive human normal diploid fibroblasts did not show a sign of AAP-2-induced apoptosis, as was examined by DAPI staining (see above).

In conclusion, we have identified an Apoptin-associating protein, namely AAP-2, which has a tumor-specific preference for induction of apoptosis and nuclear accumulation.

### Further sequence analysis

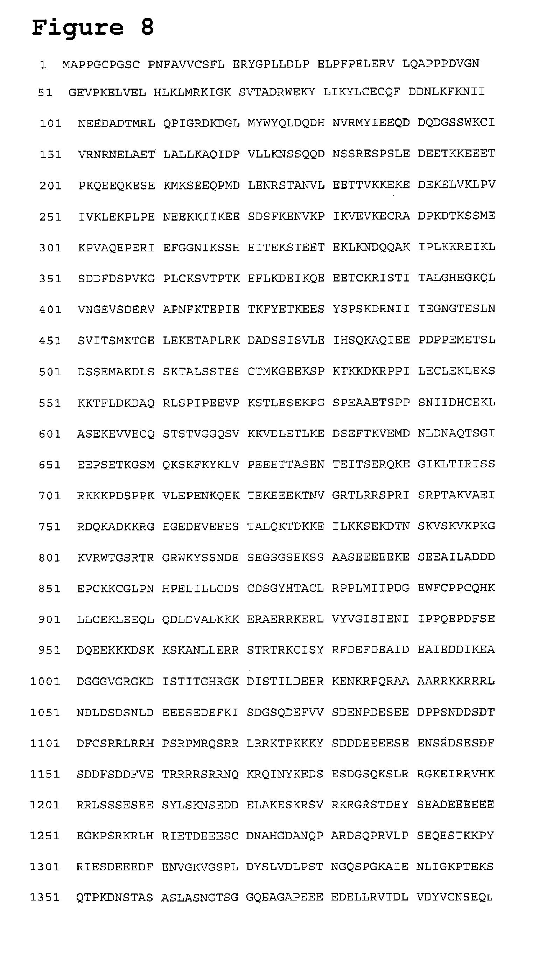

A further sequence analysis of the human AAP-2 nucleic acid sequence yielded the 5331bp long nucleic acid sequence given in figure 7. An open reading frame was found in this nucleic acid sequence at position 300-4499. The deduced amino acid sequence is given in figure 8.

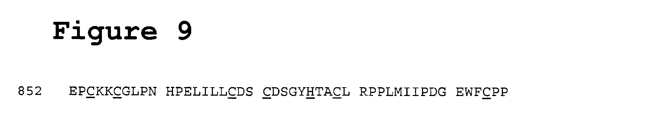

A protein domain called PHD-finger was found in the amino acid sequence of the human AAP-2 protein. It spans the region of amino acid 852 to amino acid 900. The Cys₄-His-Cys₃ zinc-finger-like motif which is characteristic for a PHD-finger domain (R. Aasland et al., 1995, TIBS 20, 56-59) is found in said region (see figure 9).

The PHD-finger is found in nuclear proteins thought to be involved in chromatin-mediated transcriptional regulation. The PHD-finger was originally identified in a set of proteins that includes members of the Drosophila Polycomb and trithorax group genes. These genes regulate the expression of the homeotic genes through a mechanism thought to involve some aspect of chromatin structure. Other proteins which have this motif also have additional domains or characteristics that support that suggestion that the PHD-finger is involved in chromatin-mediated gene regulation. PHD-fingers are thought to be protein-protein interaction domains. Such protein-protein interactions are important for e.g. the activity of multicomponent complexes involved in transcriptional activation or repression. PHD-fingers may also recognise a family of related targets in the nucleus such as the nucleosomal histone tails. (R. Aasland et al., 1995, TIBS 20, 56-59).
PHD-finger domains are also found in a number of proteins closely associated with human tumorigenisis such as HRX/ALL1/MLL/Htrx, CBP, MOZ, all of which are part of aberrant fusion proteins derived from chromosomal translocations found in a high percentage of human leukemias (for review see Jacobson and Pillus, 1999, Current Opinion in Genetics & Dev. 9, 175-184). Other PHD-finger domain-containing proteins are overexpressed in certain tumor types (Lu, P.J. et al, 1999, J.Biol. Chem. 274, 15633-45).

Therefore, interfering with the functional activity of the PHD-finger domain of AAP-2 should have therapeutic effects against human tumors. The PHD-finger domain can be used to identify substances which bind to the PHD-finger domain. This can be done by methods known to persons skilled in the art, e.g. by binding studies, where an AAP-2 peptide comprising the PHD-finger domain is bound to a matrix and it is tested whether test substances bind to the AAP-2 peptide, or by co-immunoprecipitation of an AAP-2 peptide comprising the PHD-finger domain with test substances using antibodies generated against the AAP-2 peptide comprising the PHD-finger domain. Test substances may be small organic compounds derived e.g. from a compound library or peptides or proteins derived e.g. from a peptide library or from a natural source like a cell extract. The test substances may be labeled for easier detection. The substances found to bind to the PHD-finger domain may either enhance or inhibit one or more effects of AAP-2. This can be tested by measuring the apoptotic activity of AAP-2 as described above in the presence of said substances and by determining the nuclear localization of AAP-2 as described above in the presence of said substances.

### Another Apoptin-associating protein.

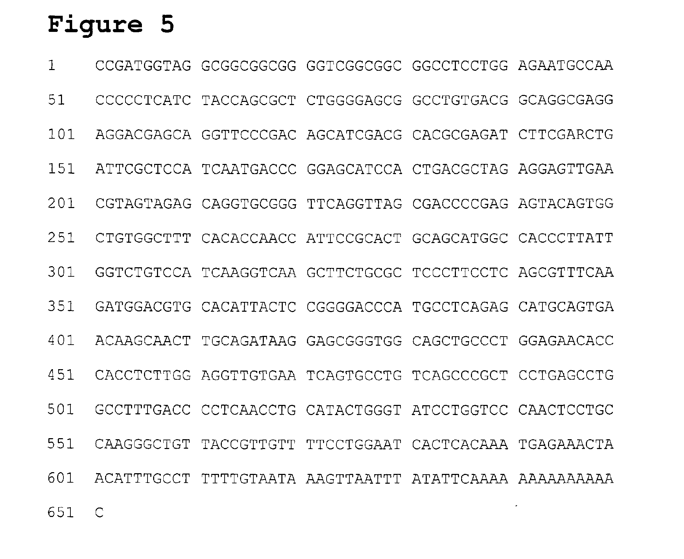

The genetic yeast screen with pGBT-VP3 as bait plasmid and pACT plasmid containing cDNAs from transformed human B cells also delivered the novel gene Apoptin-associating protein 3 (AAP-3). The DNA sequence of the AAP-3 is shown in figure 5, whereas the AAP-3 cDNA-encoded amino-acid sequence is shown in figure 6.
To analyze into further detail the associating properties of Apoptin and this AAP-3 protein, we have expressed a Myc-tagged AAP-3 cDNA by means of the pSM2NT vector (as described for AAP-2) in transformed mammalian Cos cells. Western blot analysis using the Myc-tag-specific antibodies 9E10 showed a specific (Myc-tagged) AAP-3 protein of approximately 22-kDa. This major 22-kDa AAP-3 product is accompanied by smaller and larger minor AAP-3-specific products. These results indicate that the isolated cDNA indeed encodes a protein of the expected size.
Next, immunoprecipitation assays were carried out with transiently transfected Cos cells co-synthesizing Myc-tagged AAP-3 and Apoptin. The results clearly showed that both 9E10 antibodies and Apoptin-specific 111.3 antibodies precipitate AAP-3 protein and Apoptin, which indicates that Apoptin associates with this new AAP-3 protein in a mammalian transformed background. In total, three independent immunoprecipitation experiments were carried out, which all showed the associating ability of Apoptin to the AAP-3 protein.
Immunofluorescence assays of human transformed Saos-2 cells and normal diploid VH10 fibroblasts expressing AAP-3 revealed that AAP-3 is located in both cell types predominantly in the cytoplasm and nucleus, but in lower percentages also mainly in the nucleus or mainly in the cytoplasm.. Co-synthesis of AAP-3 and Apoptin in both cell types showed a clear peri-nuclear co-localization of AAP-3 and Apoptin. Tumor cells that have become apoptotic showed a nuclear localization of Apoptin and a peri-nuclear stainings pattern of AAP-3. As expected, normal diploid VH10 cells synthesizing both Apoptin and AAP-3 did not undergo apoptosis.
These data indicate that AAP-3 will release Apoptin when the cell has become tumorigenic and/or transformed, resulting in the nuclear localization of Apoptin and induction of apoptosis.
In summary, our findings prove that our newly discovered AAP-3 protein is able to associate to the tumor-specific apoptosis-inducing protein Apoptin in both a yeast and mammalian cellular background. Therefore, this AAP-3 protein plays an important role in the induction of (Apoptin-regulated) tumors-specific apoptosis.

### Utility of AAP-2 and AAP-3 antisera

The best AAP-2 and AAP-3 antibody preparations obtained from the two rabbit derived antisera were selected for further use. We tested the efficacy of these antibody preparations against AAP-2 and AAP-3 respectively, by transfecting primate COS-7 and human U₂OS cells with the AAP-2-myc or AAP-3-myc construct. Western analysis showed that the approximately 70 kDa AAP-2-myc protein and the approximately 22 kDa AAP-3-myc were detected strongly only in samples where the DNA was transfected. Similarly, in immunoprecipitation experiments, AAP-2-myc or AAP-3-myc protein was also strongly detected. Finally, localization of AAP-2-myc or AAP-3-myc protein in a cell using the AAP-2 or AAP-3 antibody could be determined by immunofluorescence analysis.

### Overexpression of 2 Apoptin-associating proteins

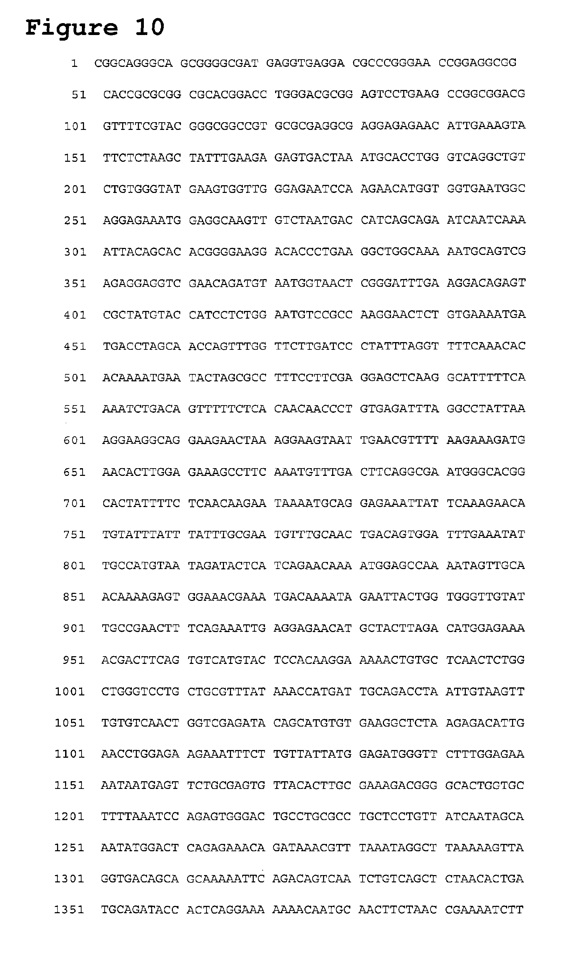

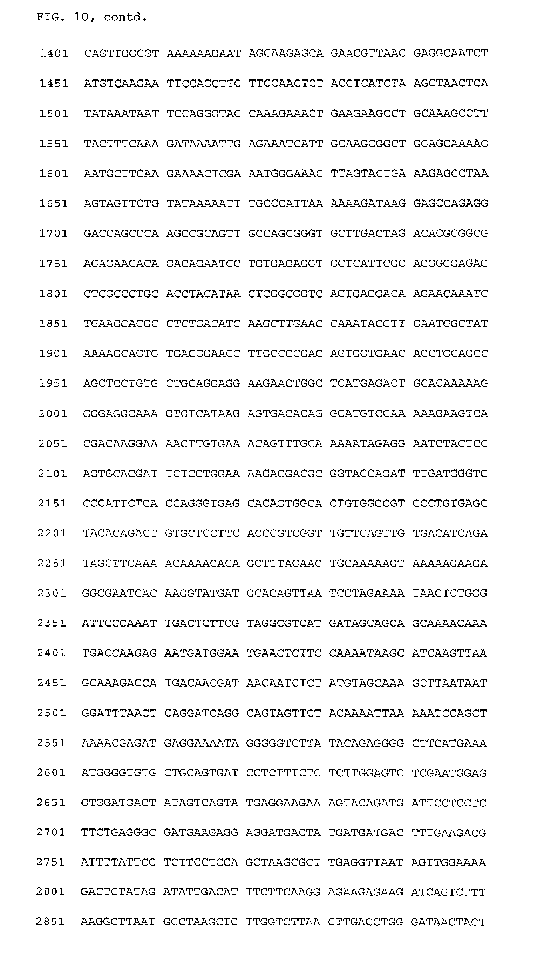

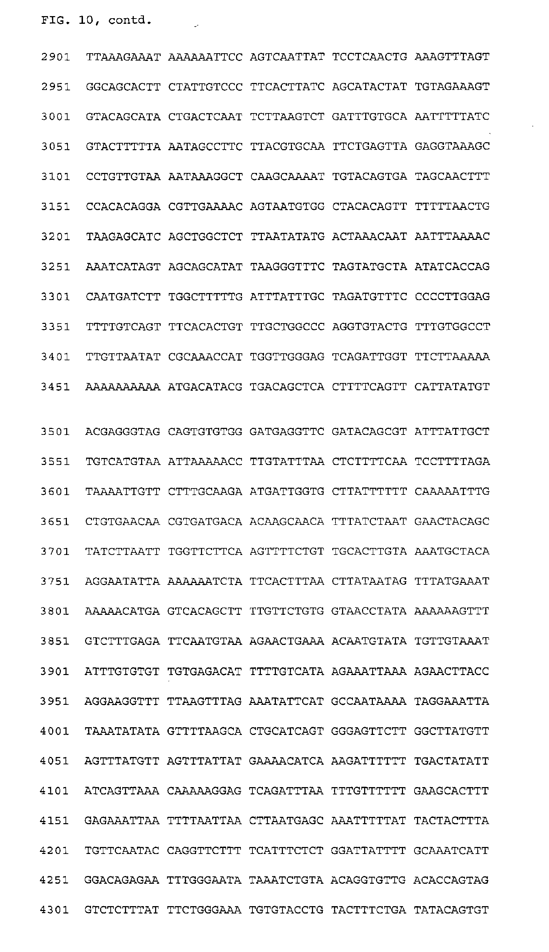

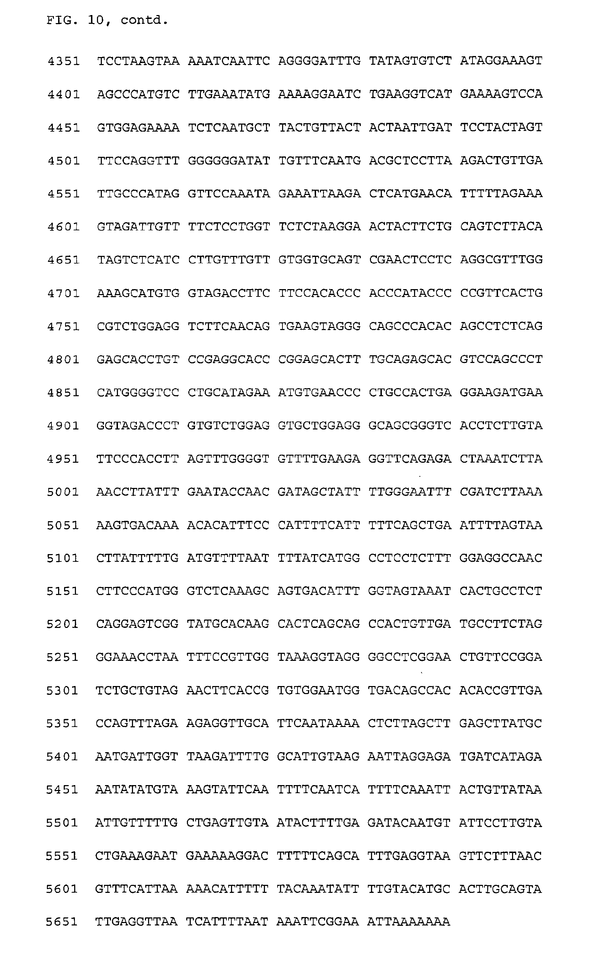

The genetic yeast screen with pGBT-VP3 as bait plasmid and pACT plasmid containing cDNAs from transformed human B cells also delivered another gene, which also encodes an Apoptin-associating protein. This Apoptin-associating protein was called AAP-4 (see co-pending application EP00204396.6, which is incorporated herein by reference). The nucleic acid sequence of AAP-4 is shown in Figure 9. An open reading frame was found in this nucleic acid sequence at position 236 to 2866. The deduced amino acid sequence is given in Figure 10. Just like AAP-2 and AAP-3, AAP-4 is able to associate with Apoptin not only in the yeast background, but also in a mammalian transformed cellular system. Furthermore, this protein is present in the nucleus and able to induce (p53-independent) apoptosis in human tumor cells. A functional equivalent or a functional fragment of AAP-4 is herein also included. A functional equivalent or a functional fragment of AAP-4 is a derivative or a fragment having the same kind of activity possibly in different amounts. It is clear to a person skilled in the art that there are different ways of arriving at a functional equivalent or functional fragment. A functional equivalent can for example be a point mutant or a deletion mutant or a equivalent derived from another species. Another way to arrive at a functional equivalent is a molecular evolution of equivalents and/or fragments having the same kind of activity possibly in different amounts.
To study whether two separate Apoptin-associating proteins can not only bind to Apoptin but also to another Apoptin-associating protein, we carried out the following experiment.

Immunoprecipitation assays were carried out with transiently transfected Cos cells co-synthesizing Myc-tagged AAP-3 and Myc-tagged AAP-4. The results clearly showed that antibodies directed against AAP-3 and antibodies directed against AAP-4 both precipitate AAP-3 and AAP-4, which suggests that AAP-3 and AAP-4 associate in this mammalian transformed background. In total, three independent immunoprecipitation experiments were carried out, which all showed the associating ability of AAP-3 and AAP-4.
The fact that two proteins, which showed to be Apoptin-associating proteins can independently co-associate in the absence of Apoptin strengthens the idea that the AAP-3/Apoptin co-association is physiologically relevant.

### Diagnostic assay for cancer cells.

Based on the present report, we can conclude that the cellular localization of AAP-2 is different in tumorigenic/transformed human cells in comparison to normal human non-transformed cells. Furthermore, accumulation of AAP-2 in the nucleus correlates with apoptosis induction, whereas cytoplasmic/nuclear localization correlates with cell viability and normal proliferative capacity. Therefore, we are able to develop a diagnostic assay for the identification of (human) cancer cells versus normal "healthy" non-transformed cells.

The assay consists of transfecting "suspicious" (human) cells, for instance from human origin, with a plasmid encoding AAP-2, or infecting the cells with viral vectors expressing AAP-2. Subsequently, the cells will be examined, 1) for the ability to undergo apoptosis by the over-expressing AAP-2 gene and 2) for a main shift in the localization of AAP-2 from the cytoplasm to the nucleus.
The intracellular localization of AAP-2 can be determined, using an immunofluorescence assay with monoclonal antibodies specific for AAP-2 and/or specific for a tag linked to AAP-2 such as the herein described myc-tag. If the percentage of apoptosis and/or the nuclear localization of AAP-2 in the analyzed cells expressing AAP-2 is significantly higher than in AAP-2-positive control "healthy" cells, one can conclude that the analyzed cells has become tumorigenic/transformed. As positive control known human tumorigenic cells will be used for expressing AAP-2.

### Co-expression of SV40 large T antigen and AAP-2 results in translocation of AAP-2 and induction of apoptosis.

We have examined the effect of expression of transforming genes on AAP-2-induced apoptosis in normal human cells derived from healthy individuals. To that end, human VH10 diploid fibroblasts were transiently co-transfected with plasmid pMT2SM-AAP-2 encoding AAP-2 protein and either plasmid pR-s884 encoding SV40 large T antigen, or the negative-control plasmid pCMV-neo (Noteborn and Zhang, 1998).
By indirect immunofluorescence, the cells were analyzed for AAP-2-induced apoptosis. The normal VH10 cells did not undergo apoptosis when AAP-2 was transfected with the negative-control plasmid. The results showed, as expected, that expression of AAP-2 is not able to induce apoptosis in normal human diploid cells, confirming the above mentioned data. However, normal diploid human fibroblasts expressing both AAP-2 and SV40 large T antigen underwent AAP-2-induced apoptosis.
The transition of normal human cells, from AAP-2-resistance to AAP-2-susceptibility, can probably be explained by the fact that the AAP-2 protein translocates from a cytoplasmic localization to a nuclear localization. This transition becomes apparent already 2 days after transfection of plasmids encoding the transforming protein SV40 large T antigen. One can conclude that an event takes place, in this example due to expression of a transforming product derived from a DNA-tumor virus, which results in the translocation of over-expressed AAP-2 from the cytoplasm to the nucleus, which is followed by induction of apoptosis.

### Diagnostic assay for cancer-inducing genes, agents and cancer- proneness based on AAP-2-induced apoptosis.

Based on the present report, we are able to develop a diagnostic assay for the identification of cancer-inducing and/or transforming agents or genes.
A first type of assay consists of transfecting "normal" cells, for instance from human origin, with a plasmid encoding AAP-2, or infecting the cells with viral vectors expressing AAP-2, together with a plasmid encoding a putative transforming/cancer-inducing gene. Subsequently, the cells will be examined, 1) for the ability to undergo apoptosis by the over-expressing AAP-2 gene and 2) for a shift in the localization of AAP-2 from the cytoplasm to the nucleus.
The intracellular localization of AAP-2 can be determined, using an immunofluorescence assay with monoclonal antibodies specific for AAP-2 and/or specific for a tag linked to AAP-2 such as the herein described myc-tag. If the percentage of apoptosis and/or the nuclear localization of AAP-2 in normal cells co-expressing AAP-2 and the putative transforming/cancer-inducing gene is significantly higher than in AAP-2-positive control cells expressing a control plasmid, one can conclude that the analyzed gene indeed has transforming/cancer-inducing activity.
A second example of a diagnostic test is based on the treatment of cultured normal diploid cells with a putative carcinogenic agent. The agent can be added, for instance, to the culture medium for various lengths of time. Subsequently, the cells are transfected with a plasmid encoding AAP-2. This approach can also be carried out by first transfecting/infecting the normal diploid cells, and then treating the cells with the agent to be tested. The subsequent steps of the assay are the same as described for the in this section described first type of diagnostic assay. If the percentage of apoptosis and/or the nuclear localization of AAP-2 in normal cells expressing AAP-2 and the putative carcinogenic agent is significantly higher than in AAP-2-positive control cells expressing a control agent, one can conclude that the analyzed agent indeed has transforming/cancer-inducing activity.
A third example of a diagnostic test is based on the treatment of cultured normal diploid cells derived from a skin biopsy of the potential cancer-prone individual to be tested and cultured in suitable medium. Next, the cells are irradiated with UV and subsequently transfected with a plasmid encoding AAP-2, or infected with a viral vector expressing AAP-2, or the cells are first transfected and/or infected and then irradiated. In parallel, diploid cells from a normal healthy individual will be used as control.
The subsequent steps of the assay are the same as described for the in this section described first type of diagnostic assay. If after UV-treatment the percentage of apoptosis and/or the nuclear localization of AAP-2 in diploid cells derived from the potential cancer-prone individual is significantly higher than in UV-treated AAP-2-positive control cells, one can conclude that the analyzed cells are cancer-proneness ones.

### Description of the figures

Figure 1 shows the partial sequence of vector pMT2SM-AAP-2-I.

Figure 2 shows the partial sequence of vector pMT2SM-AAP-2-II.

Figure 3 shows the amino-acid sequence of the analyzed region of the Apoptin-associating clone AAP-2-II. In addition, the three C-terminal amino acids H-E-G (bold) of the multiple cloning site of pACT are given to illustrate that the AAP-2 amino acid sequence is in frame with the GAL4-activation domain. This feature proves that the AAP-2-II region is indeed synthesized in yeast cells.

Figure 4 shows the apoptotic activity of AAP-2 protein and/or Apoptin in human osteosarcoma-derived Saos-2 cells. (-): no apoptotic activity; (+): apoptotic activity; (++): strong apoptotic activity; (+++): very strong apoptotic activity. In total three independent experiments have been carried out.

Figure 5 shows the partial sequence of vector pMT2SM-AAP-3.

Figure 6 shows the amino-acid sequence of the analysed region of the Apoptin-associating clone AAP-3. In addition, the three C-terminal amino acids H-E-G (bold) of the multiple cloning site of pACT are given to illustrate that the AAP-3 amino acid sequence is in frame with the GAL4-activation domain. This feature proves that the AAP-3 region is indeed synthesized in yeast cells.

Figure 7 shows the nucleic acid sequence of AAP-2.

Figure 8 shows the amino acid sequence deduced from the nucleic acid sequence of figure 7

Figure 9 shows the PHD-finger domain of the AAP-2 protein.

Figure 10 shows the nucleic acid sequence of AAP-4.

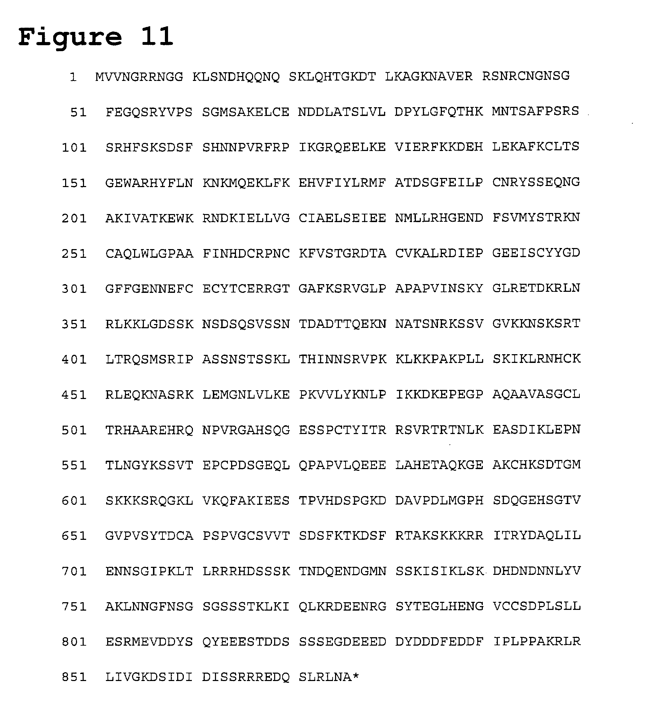

Figure 11 shows the amino acid sequence deduced from the nucleic acid sequence of figure 10.

### REFERENCES

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
3. Danen-Van Oorschot, A.A.A.M, Den Hollander, A., Takayama, S., Reed, J., Van der Eb, A.J. and Noteborn, M.H.M. (1997a). BAG-1 inhibits p53-induced but not Apoptin-induced apoptosis. Apoptosis 2, 395-402.
4. Duke, R.C., Ocjius, D.M., Young, J, D-E. (1996). Cell suicide in health and disease. Scientific American December 1996, 48-55.
5. Durfee, T., Becherer, K., Chen, P.-L., Yeh,S.-H., Yang, Y., Kilburn, A.E., Lee, W.-H., and Elledge, S.J. (1993). The retinoblastoma protein associates with the protein phosphate type I catalytic subunit. Genes and Development 7, 555-569.
6. Earnshaw, W.C., 1995. Nuclear changes in apoptosis. Current Opinion in Cell Biology 7, 337-343.
7. Fields, S. and Song, O.K. (1989). A novel genetic system to detect protein-protein interactions. Nature 340, 245-246.
8. Hockenberry, D.M. (1994). Bcl-2 in cancer, development and apoptosis. Journal of Cell Science, Supplement 18, 51-55.
9. Hoffman, C.S. and Winston, F. (1987). A ten-minute DNA preparation from yeast efficiently releases autonomous plasmids for transformation of Escherichia coili. Gene 57, 267-272.
10. Kerr, J.F.R., Winterford, C.M., and Harmon, B.V. (1994). Apoptosis: Its significance in cancer and cancer therapy. Cancer 73, 2013-2026.
11. Klebe, R.J., Harriss, J.V., Sharp, Z.D., and Douglas, M.G. (1983). A general method for polyethylene-glycol-induced genetic transformation of bacteria and yeast. Gene 25, 333-341.
12. Levine, A.J. (1997). p53, the cellular gatekeeper for growth and division. Cell 88, 323-331.
13. Maniatis, T., Fritsch, E.F., and Sambrook, J. (1982). Molecular Cloning: A Laboratory Manual. CSHL Press, New York, USA.
14. McDonell T.J., Meyn, R.E., Robertson, L.E. (1995). Implications of apoptotic cell death regulation in cancer therapy. Seminars in Cancer Biology 6, 53-60.
15. Noteborn, M.H.M. (1996). PCT application WO 96/41191. Apoptin induces apoptosis in human transformed and malignant cells but not in normal cells as essential characteristic for the development of an anti-tumor therapy.
16. Noteborn, M.H.M., and De Boer, G.F. (1996). Patent USA/no. 030, 335.
17. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
18. Noteborn, M.H.M., and Pietersen, A. (1998). A gene delivery vehicle expressing the apoptosis-inducing proteins VP2 and/or Apoptin. PCT Application no. PCT/NL98/00213
19. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
20. Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology, 79, 3073-3077.
21. Noteborn, M.H.M., and Zhang, Y. (1998). Methods and means for determining the transforming capability of agents, for determining the predisposition of cells to become transformed and prophylactic treatment of cancer using Apoptin-like activity. PCT Application no. PCT/NL98/00457
22. Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
23. Paulovich, A.G., Toczyski, D., Hartwell, H. (1997). When checkpoints fail. Cell 88, 315-321.
24. Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1999). Specific tumor-cell killing with adenovirus vectors containing the Apoptin gene. Gene Therapy 6, 882-892.
25. Rose, M.D., Winston, F., and Hieter, P. (1990). Methods in yeast genetics. A laboratory course manual. Cold Spring Harbor Laboratory Press, Cold Spring Harbor, New York, USA.
26. Sachs, L. and Lotem, J. (1993). Control of programmed cell death in normal and leukemia cells: New implications for therapy. Blood 82, 15-21.
27. Sanger, F., Nicklen, S., and Coulsen, A.R. (1977). DNA sequencing with chain-terminating inhibitors. Proceedings National Academic Sciences USA 74, 5463-5467.
28. Steller, H. (1995). Mechanisms and genes of cellular suicide. Science 267, 1445-1449.
29. Telford, W.G., King, L.E., Fraker, P.J. (1992). Comparative evaluation of several DNA binding dyes in the detection of apoptosis-associated chromatin degradation by flow cytometry. Cytometry 13, 137-143.
30. Teodoro, J.G. and Branton, P.E. (1997). Regulation of apoptosis by viral gene products. Journal of Virology 71, 1739-1746.
31. Thompson, C.B. (1995). Apoptosis in the pathogenesis and treatment of disease. Science 267, 1456-1462.
32. White, E. (1996). Life, death, and the pursuit of apoptosis. Genes and development 10, 1-15.
33. Wyllie, A.H. (1995). The genetic regulation of apoptosis. Current Opinion in Genetics and Development 5, 97-104.
34. Wyllie, A.H., Kerr, J.F.R., Currie, A.R. (1980). Cell death: The significance of apoptosis. International Review of Cytology 68, 251-306.
35. Yang, X., Hubbard, E.J.A., and Carlson, M. (1992). A protein kinase substrate identified by the two-hybrid system. Science 257, 680-682.
36. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells *in vitro.* Leukemia 9 S1, 118-120.
37. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.
38. R. Aasland et al., 1995, TIBS 20, 56-59
39. Jacobson and Pillus, 1999, Current Opinion in Genetics & Dev. 9, 175-184
40. Lu, P.J. et al, 1999, J.Biol. Chem. 274, 15633-45
41. Evan GI, Lewis GK, Ramsay G, Bishop JM, (1985), Isolation of monoclonal antibodies specific for human c-myc proto-oncogene product. Mol. Cell. Biol. Dec 5 (12), 3610-3616
42. Noteborn et al (2000) European patent application 00204396.6, Apoptin-associating protein

## Claims

1. An isolated or recombinant nucleic acid or functional equivalent or functional fragment thereof encoding an Apoptin-associating proteinaceous substance capable of providing apoptosis.

2. A nucleic acid according to claim 1 derived from a cDNA library.

3. A nucleic acid according to claim 1 or 2 wherein said cDNA library comprises human cDNA.

4. A nucleic acid according to anyone of claims 1 to 3 capable of hybridising to a nucleic acid molecule encoding an Apoptin-associating proteinaceous substance as shown in figure 1, 2, 5 or 7.

5. A nucleic acid according to anyone of claims 1 to 4 being at least 60% homologous to a nucleic acid molecule, or to a functional equivalent or functional fragment thereof, encoding an Apoptin-associating proteinaceous substance as shown in figure 1, 2, 5 or 7.

6. A vector comprising a nucleic acid according to anyone of claims 1 to 5.

7. A vector according to claim 6 comprising a gene-delivery vehicle.

8. A host cell comprising a nucleic acid according to anyone of claims 1 to 5 or a vector according to claim 6 or 7.

9. A host cell according to claim 8 which is a eukaryotic cell such as a yeast cell or a vertebrate cell.

10. An isolated or recombinant Apoptin-associating proteinaceous substance capable of providing apoptosis.

11. A proteinaceous substance according to claim 10 encoded by a nucleic acid according to anyone of claims 1 to 5.

12. A proteinaceous substance according to claim 10 or 11 comprising at least a part of an amino acid sequence as shown in figure 3, 6 or 8 or a functional equivalent or functional fragment thereof.

13. An isolated or synthetic antibody specifically recognising a proteinaceous substance or functional equivalent or functional fragment thereof according to anyone of claims 10 to 12.

14. A proteinaceous substance specifically recognisable by an antibody according to claim 13.

15. Use of a nucleic acid according to anyone of claims 1 to 5, a vector according to claims 6 or 7, a host cell according to claim 8 or 9, a proteinaceous substance according to anyone of claims 10 to 12 or 14 for the induction of apoptosis.

16. Use according to claim 15 wherein said apoptosis is p53-independent.

17. Use according to claim 15 or 16 further comprising use of a nucleic acid encoding Apoptin or a functional equivalent or fragment thereof or use of Apoptin or a functional equivalent or fragment thereof.

18. A pharmaceutical composition comprising a nucleic acid according to anyone of claims 1 to 5, a vector according to claims 6 or 7, a host cell according to claim 8 or 9, a proteinaceous substance according to anyone of claims 10 to 12 or 14.

19. A pharmaceutical composition according to claim 18 further comprising a nucleic acid encoding Apoptin or a functional equivalent or fragment thereof or Apoptin or a functional equivalent or fragment thereof.

20. A pharmaceutical composition according to claim 18 or 19 for the induction of apoptosis.

21. A pharmaceutical composition according to claim 20 wherein said apoptosis is p53-independent.

22. A pharmaceutical composition according to anyone of claims 18 to 21 for the treatment of a disease where enhanced cell proliferation or decreased cell death is observed.

23. A pharmaceutical composition according to claim 22 wherein said disease comprises cancer or auto-immune disease.

24. A method for treating an individual carrying a disease where enhanced cell proliferation or decreased cell death is observed comprising treating said individual with a pharmaceutical composition according to anyone of claims 18 to 23.

25. A method for detecting the presence of cancer cells or cells that that are cancer prone in a sample of cells comprising transfecting cells in said sample with a nucleic acid according to anyone of claims 1 to 5, or a vector according to claims 6 or 7, and determining the percentage of apoptosis of cells in said sample.

26. A method for detecting the presence of cancer cells or cells that that are cancer prone in a sample of cells comprising transfecting cells in said sample with a nucleic acid according to anyone of claims 1 to 5, or a vector according to claims 6 or 7, and determining the intracellular localisation of a proteinaceous substance derived from said nucleic acid or vector in cells in said sample.

27. A method according to claim 26 therein the presence of said proteinaceous substance in said cells is detected by immunostaining said cells with an antibody.

28. A method according to claim 27 wherein said antibody comprises an antibody according to claim 13.

29. A method for identifying a putative cancer-inducing agent comprising submitting a sample of cells to said agent, and detecting the presence of cancer cells or cells that are cancer prone in a sample of cells with a method according to anyone of claims 25 to 28.

30. A method according to claim 29 wherein said putative cancer-inducing agent comprises a genome or functional fragment thereof.

31. An isolated or recombinant nucleic acid as set forth in SEQ ID No 6.

32. An isolated or recombinant proteinaceous substance encoded by a nucleic acid as set forth in SEQ ID No 6.

33. An isolated or recombinant proteinaceous substance comprising the amino acid sequence as set forth in SEQ ID No 7.

34. An isolated or recombinant proteinaceous substance comprising the amino acid sequence as set forth in SEQ ID No 8.

35. An assay to identify a putative effector of the activity of the proteinaceous substance encoded by a nucleic acid as set forth in SEQ ID No 6 comprising bringing in contact a proteinaceous substance comprising the amino acid sequence as set forth in SEQ ID No 8 with said effector and determining the binding of said effector.
